(19) Europäisches Patentamt / European Patent Office / Office européen des brevets

(11) **EP 4 073 176 B1**

(12) **EUROPEAN PATENT SPECIFICATION**

(45) Date of publication and mention
of the grant of the patent:
**20.08.2025 Bulletin 2025/34**

(21) Application number: **20824812.0**

(22) Date of filing: **19.11.2020**

(51) International Patent Classification (IPC):
*C09C 1/00* (2006.01)   *A61K 8/06* (2006.01)
*A61K 8/26* (2006.01)   *A61K 8/29* (2006.01)
*A61K 8/34* (2006.01)   *A61K 8/42* (2006.01)
*A61K 8/891* (2006.01)   *A61Q 1/06* (2006.01)
*C09C 1/36* (2006.01)

(52) Cooperative Patent Classification (CPC):
**C09C 1/0081; A61K 8/064; A61K 8/26; A61K 8/29;
A61K 8/345; A61K 8/42; A61K 8/891; A61Q 1/06;
C09C 1/0084;** A61K 2800/43; A61K 2800/432;
A61K 2800/612; C01P 2004/62; C01P 2006/60;
C01P 2006/62;                                    (Cont.)

(86) International application number:
**PCT/US2020/061221**

(87) International publication number:
**WO 2021/118781 (17.06.2021 Gazette 2021/24)**

(54) **COMPOSITIONS WITH INCREASED COLOR SHADE STABILITY BASED ON PIGMENTARY TIO2, ORGANIC PIGMENTS AND METAL OXIDE PARTICLES**

ZUSAMMENSETZUNGEN MIT ERHÖHTER FARBTONSTABILITÄT BASIEREND AUF PIGMENTIERTEM TIO2, ORGANISCHEN PIGMENTEN UND METALLOXIDPARTIKELN

COMPOSITIONS À STABILITÉ DE TEINTE ACCRUE À BASE DE TIO2 PIGMENTAIRE, DE PIGMENTS ORGANIQUES ET DE PARTICULES D'OXYDE MÉTALLIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.12.2019 US 201962946845 P**

(43) Date of publication of application:
**19.10.2022 Bulletin 2022/42**

(73) Proprietor: **Natura & Co UK Holdings Limited
Bristol, BS1 6AJ (GB)**

(72) Inventors:
• **HUTSON, Ashley L.
Florida, New York 10921 (US)**
• **HUANG, Gloria
Lincoln Park, New Jersey 07035 (US)**

(74) Representative: **Abel & Imray LLP
Westpoint Building
James Street West
Bath BA1 2DA (GB)**

(56) References cited:
WO-A1-2019/241427   WO-A2-2014/158599
US-A- 4 795 631   US-A1- 2007 020 209

(52) Cooperative Patent Classification (CPC): (Cont.)
C01P 2006/63; C01P 2006/64; C09C 1/3669

**Description**

**FIELD OF INVENTION**

The present invention relates generally to methods and compositions for topical application to human integuments, including skin and lips. More specifically, the present invention relates to compositions with increased color stability, and methods of using these compositions.

**BACKGROUND OF INVENTION**

Pigmented topical products are often created using either emulsions or anhydrous systems. It is common to include materials such as iron oxide pigments in hydrous systems to create varying colors of skin-tone. Usually, anhydrous systems consist predominantly of oils and waxes and are used for formulating eyeshadows and lipsticks. These anhydrous systems often contain a chemical class of dyes called azo dyes allowing for the creation of compositions in a large range of the color palette. When these azo dyes are precipitated as salts onto a substrate they become brightly colored lake pigments which are identified by the D&C and FD&C colorant classes.

Cosmetic compositions are known, for example, from WO 2014/158599 A2 which discloses cosmetic compositions comprising a gel formed from a cellulosic polymer and a polar oil, a non-polar film-forming agent, polyhydroxystearic acid, and one or more pigments. US 2007/0020209 A1 discloses emulsion makeup compositions for keratinous surfaces which change color upon application and methods for treating keratinous surfaces with an emulsion cosmetic composition that changes color upon application. US 4,795,631 discloses water-resistant lip-films formed from water-based compositions comprising water; an alkali-dispersible or alkali soluble, water-insoluble thermoplastic film forming resin; a volatile base; and a water-insoluble plasticizer.

Typically, D&C and FD&C lake pigments cannot be incorporated into hydrous systems due to hydrolysis at several sites within these large molecules. It is well known that treating the lake pigments can retard the hydrolysis in hydrous systems to some extent. For example, ITT-treatment of organic pigments is often used to increase the color stability of compositions comprising azo dyes. However, factors such as heat during the manufacturing and filling processes can counteract the pigment treatment and cause the azo dye based colorants to hydrolyze regardless of surface treatment. Such hydrolysis will alter the resultant color of compositions over the course of manufacture thus resulting in a less desired product. Instead of organic pigments, iron oxides are often used in conjunction with titanium dioxide to create a muted palette of shades.

Cosmetic products also often use titanium dioxide ("$TiO_2$") which is mined as a combination of rutile and anatase crystalline forms. For example, WO 2019/241427 A1 (which forms part of the state of the art only for the purposes of Article 54(3) EPC) relates to compositions comprising organic pigments and rutile $TiO_2$ and EP 2,724,985 A1 discloses a cosmetic containing rutile-type titanium dioxide. Anatase is the predominantly used form of $TiO_2$ in color cosmetics. The titanium dioxide is combined with organic pigment materials to yield brighter colors seen in lipsticks, blush, and eyeshadow by providing increased refraction of pigment colors ($TiO_2$ is typically a white solid). However, electrons donated from titanium dioxide to the system often exacerbate the color shade shift of compositions-in particular in aqueous compositions with organic pigments. Accordingly, true emulsion lipsticks do not exist in a full shade palette of bright colors since the colors accessed by various combinations of organic pigments are inaccessible in these lipsticks.

It is therefore an object of this invention to provide pigmented compositions comprising with reduced color shift over time.

**SUMMARY**

In accordance with the foregoing objectives and others, this present disclosure provides compositions with reduced color shift and/or homogenous color distribution. The reduction in color shift and/or homogenous color distribution may be observed following heating of the compositions for prolonged periods of time *(e.g.,* one hour, two hours, three hours, four hours, five hours, six hours, seven hours, eight hours) at elevated temperatures *(e.g.,* above 60°C). Without wishing to be bound by theory, it is believed that metal oxide components are able to prevent hydrolysis of organic components thereby conferring color stability. By stabilizing the organic pigments, the compositions of the present disclosure may be capable of being formulated in a wider shade palette than previously available. The reduced color shift is accomplished through the use of metal oxide components having a particle size of less than 50 $\mu$m or less than 25 $\mu$m or less than 10 $\mu$m or less than 1 $\mu$m. The particle size of these components may be measured by dynamic light scattering. In certain embodiments, color stability is most pronounced in aqueous systems which are heated and maintained at higher temperatures *(e.g.,* above 60°C, above 70°C, above 80°C, above 85°C) for longer periods of time *(e.g.,* eight hours) as the organic pigment instability reaction is exothermic. In particular, these higher temperatures are above the melting point of a composition base *(i.e.,* the composition without the pigment grind) where the full phase transition from solid to liquid occurs.

Provided is a pigmented composition according to claim 1. The pigmented composition may comprise:

(a) optionally surface treated pigmentary titanium dioxide;

(b) optionally surface treated organic pigments; and

(c) an optionally surface treated metal oxide particles having an average particle size of less than 50 $\mu$m (*e.g.,* as measured by dynamic light scattering);

wherein the weight ratio of said pigmentary titanium dioxide and said organic pigments to said metal oxide particles in said composition is greater than 1:1 (*e.g.,* 100:1 to 1:1, 50:1 to 1:1, 30:1 to 1:1, 100:1 to 2:1, 50:1 to 2:1, 30:1 to 2:1, 50:1 to 1:1, 100:1 to 5:1, 50:1 to 5:1, 30:1 to 5:1), and wherein said metal oxide particles are selected from silica, alumina, zirconia, zinc oxide, indium tin oxide, ceria, and mixtures thereof, and/or wherein said metal oxide particles are fumed metal oxide particles.

Typically, the compositions are in the form of an emulsion. The emulsion may be, for example, a water-in-oil, oil-in-water, silicone-in-water, water-in-silicone, polyol-in-oil, oil-in-polyol, glycerin-in-oil, oil-in-glycerin, silicone-in-glycerin, glycerin-in-silicone, silicone-in-polyol, or polyol-in-silicone emulsion. In one embodiment, the emulsion is a water-in-oil, oil-in-water, glycerin-in-oil, silicone-in-water, or water-in-silicone emulsion. In further embodiments, the emulsion is a glycerin-in-oil, or water-in-oil emulsion. In certain embodiments, the emulsion is a glycerin-in-oil emulsion.

Also provided is a pigmented composition in the form of a glycerin-in-oil emulsion according to claim 11. In certain implementations, the pigmented composition may be in the form of a glycerin-in-oil emulsion comprising:

(a) from 0.1% to 10% optionally surface treated rutile pigmentary TiO$_2$ by weight of the composition; and

(b) from 1% to 10% optionally surface treated organic pigments (*e.g.,* Red 7, Red 6, Red 27, Red 33, Blue 1, Yellow 5) by weight of the composition;

(c) from 0.1% to 10% optionally surface treated fumed alumina by weight of the composition;

(d) from 20% to 50% silicone by weight of the composition; and

(e) from 1% to 50% (*e.g.,* 1% to 10%, 20% to 30%, 30% to 40%) glycerin by weight of the composition;

wherein the weight ratio of said pigmentary titanium dioxide and said organic pigments to said metal oxide particles in said composition is greater than 1:1, and wherein the metal oxide particles have a particle size of less than 50 $\mu$m.

A method for coloring a human integument is also provided according to claim 14. In embodiments, the method comprises applying to the human integument a composition comprising:

(a) optionally surface treated pigmentary titanium dioxide (*e.g.,* rutile pigmentary TiO$_2$);

(b) optionally surface treated organic pigments (*e.g.,* Red 7, Red 6, Red 27, Red 33, Blue 1, Yellow 5); and

(c) an optionally surface treated metal oxide particle having an average particle size of less than 50 $\mu$m (*e.g.,* as measured by dynamic light scattering);

wherein the weight ratio of said pigmentary titanium dioxide and said organic pigments to said metal oxide particles in said composition is greater than 1:1 (*e.g.,* 100:1 to 1:1, 50:1 to 1:1, 30:1 to 1:1, 100:1 to 2:1, 50:1 to 2:1, 30:1 to 2:1, 50:1 to 1:1, 100:1 to 5:1, 50:1 to 5:1, 30:1 to 5:1), and wherein said metal oxide particles are selected from silica, alumina, zirconia, zinc oxide, indium tin oxide, ceria, and mixtures thereof, and/or wherein said metal oxide particles are fumed metal oxide particles. In some embodiments, the human integument is a keratinous surface. The keratinous surface may be hair (*e.g.,* eyebrows, eyelashes), skin, lips, or nails (*e.g.* toenails, fingernails, cuticles). In some implementations, the compositions are applied to the lips. In some embodiments, application to a human integument results in the formation of a film on the integument.

## BRIEF DESCRIPTION OF FIGURES

FIG. 1 shows the measured color change ("ΔE") after eight (8) hours of heating anhydrous and glycerin-in-oil emulsions with different pigment components.

FIG. 2 shows the color stability of several emulsion formulas as measured by changes in the L*a*b* ("ΔE") color space over time.

## DETAILED DESCRIPTION

Detailed embodiments of the present invention are disclosed herein; however, it is to be understood that the disclosed embodiments are merely illustrative of the invention that may be embodied in various forms. In addition, each of the examples given in connection with the various embodiments of the invention is intended to be illustrative, and not restrictive.

All terms used herein are intended to have their ordinary meaning in the art unless otherwise provided. All concentrations are in terms of percentage by weight of the specified component relative to the entire weight of the topical composition, unless otherwise defined.

As used herein, "a" or "an" shall mean one or more. As used herein when used in conjunction with the word "comprising," the words "a" or "an" mean one or more than one. As used herein "another" means at least a second or more.

As used herein, "consisting predominantly" with respect to the rutile content of $TiO_2$ means that the $TiO_2$ is predominantly composed of rutile-$TiO_2$. For example, compositions with $TiO_2$ consisting predominantly of rutile $TiO_2$ shall mean that the $TiO_2$ present in the composition is above 60%, 70%, 80%, or 90%, rutile $TiO_2$ by weight of all the $TiO_2$ present in the composition (*i.e.,* the $TiO_2$ component). In one embodiment, the $TiO_2$ present in the composition is above 80% by weight of the $TiO_2$ component. In a further embodiment, the $TiO_2$ present in the composition is above 90% (*e.g.,* above 95%, above 99%) by weight of the $TiO_2$ component.

As used herein, all ranges of numeric values include the endpoints and all possible values disclosed between the disclosed values. The exact values of all half integral numeric values are also contemplated as specifically disclosed and as limits for all subsets of the disclosed range. For example, a range of from 0.1% to 3% specifically discloses a percentage of 0.1%, 1%, 1.5%, 2.0%, 2.5%, and 3%. Additionally, a range of 0.1 to 3% includes subsets of the original range including from 0.5% to 2.5%, from 1% to 3%, from 0.1% to 2.5% It will be understood that the sum of all weight % of individual components will not exceed 100%.

As used herein, the term "oil" is intended to include silicone oils, unless otherwise noted. The term "oil" is intended to encompass volatile and/or nonvolatile oils. The terms "internal" and "discontinuous" phase are synonymous, as are the terms "external" and "continuous" phase. The terms "glycerin" and "glycerol" are synonymous and used interchangeably. It will be understood that the oil phases of compositions may comprise one or more silicone oils as either the primary or non-primary component of the oil phase. It will also be understood that water phases of compositions may comprise one or more polyol (*e.g.,* glycerin) as a non-primary component and polyol (*e.g.,* glycerin) phases of compositions may comprise water as a non-primary component.

The compositions of the invention are useful for application to the human integumentary system, including, skin, lips, nails, hair, and other keratinous surfaces. As used herein, the term "keratinous surface" refers to keratin-containing portions of the human integumentary system, which includes, but is not limited to, skin, lips, hair (including eyebrows and eyelashes), and nails (*e.g.,* toenails, fingernails, cuticles) of mammalians, preferably humans. A "keratin fiber" includes hair of the scalp, eyelashes, eyebrows, facial hair, and body hair such as hair of the arms, legs

The pigmented composition may comprise:

(a) optionally surface treated (*e.g.,* with isopropyl triisostearyl titanate, ITT/dimethicone) pigmentary titanium dioxide (*e.g.,* titanium dioxide consisting of or consisting predominantly of rutile phase titanium dioxide);

(b) optionally surface treated (*e.g.,* with isopropyl triisostearyl titanate, ITT/dimethicone) organic pigments; and

(c) optionally surface treated (*e.g.,* with isopropyl triisostearyl titanate, ITT/dimethicone) metal oxide particles having an average particle size of less than 50 μm (*e.g.,* as measured by dynamic light scattering);

wherein the weight ratio of said pigmentary titanium dioxide and said organic pigments to said metal oxide particles in said composition is greater than 1:1 (*e.g.,* 100:1 to 1:1, 50:1 to 1:1, 30:1 to 1:1, 100:1 to 2:1, 50:1 to 2:1, 30:1 to 2:1, 50:1 to 1:1, 100:1 to 5:1, 50:1 to 5:1, 30:1 to 5:1). In certain implementations, the composition is aqueous or comprises an aqueous phase.

The organic pigments can include, but are not limited to, at least one of carbon black, carmine, phthalocyanine blue and green pigment, diarylide yellow and orange pigments, and azo-type red and yellow pigments such as toluidine red, litho red, naphthol red and brown pigments, and combinations thereof. The organic pigment may be in salt form, for example, the Al⁺, Ba⁺, Ca⁺ salt of the organic pigment.

The compositions may comprise, for example, one or more dyes, toners or lakes. Lakes generally refer to a colorant prepared from a water-soluble organic dye (e.g., D&C or FD&C) which has been precipitated onto an insoluble reactive or adsorptive substratum or diluent. In some embodiments, the organic pigments may be azo dye based or comprise one or more azo moieties. Typically, azo based pigments are organic compounds comprising the linkage -N=N-. The term "D&C" means drug and cosmetic colorants that are approved for use in drugs and cosmetics by the FDA. The term "FD&C" means food, drug, and cosmetic colorants which are approved for use in foods, drugs, and cosmetics by the FDA. Certified D&C and FD&C colorants are listed in 21 C.F.R. § 74.101 et seq. and include the FD&C colors Blue 1, Blue 2, Green 3, Orange B, Citrus Red 2, Red 3, Red 4, Red 40, Yellow 5, Yellow 6, Blue 1, Blue 2; Orange B, Citrus Red 2; and the D&C colors Blue 4, Blue 9, Green 5, Green 6, Green 8, Orange 4, Orange 5, Orange 10, Orange 11, Red 6, Red 7, Red 17, Red 21, Red 22, Red 27, Red 28, Red 30, Red 31, Red 33, Red 34, Red 36, Red 39, Violet 2, Yellow 7, Yellow 8, Yellow 10, Yellow 11, Blue 4, Blue 6, Green 5, Green 6, Green 8, Orange 4, Orange 5, Orange 10, Orange 11, and so on. For example, the pigmented composition may comprise D&C Red 19 (e.g., CI 45170, CI 73360 or CI 45430); D&C Red 9 (CI 15585); D&C Red 21 (CI 45380); D&C Orange 4 (CI 15510); D&C Orange 5 (CI 45370); D&C Red 27 (CI 45410); D&C Red 13 (CI 15630); D&C Red 7 (CI 15850:1); D&C Red 6 (CI 15850:2); D&C Yellow 5 (CI 19140); D&C Red 36 (CI 12085); D&C Orange 10 (CI 45475); D&C Yellow 19 (CI 15985); FD&C Red 40 (CI 16035); FD&C Blue 1 (CI 42090); FD&C Yellow 5 (CI 19140); or any combinations thereof. In certain implementations, the composition may comprise one or more azo based organic pigments. For example, the pigmented composition may comprise Red 7 and/or Red 6 and/or Red 27 and/or Blue 1 and/or Yellow 5 and/or Red 33. The compositions may comprise Red 7. In certain embodiments the composition may comprise Red 7 and Red 6. In certain embodiments, the compositions may comprise Red 7 and Blue 1. In various implementations, the composition may comprise Red 7, Red 27, and Blue 1.

Substrates suitable for forming lakes include, without limitation, mica, bismuth oxychloride, sericite, alumina, aluminum, copper, bronze, silver, calcium, zirconium, barium, and strontium, titanated mica, fumed silica, spherical silica, polymethylmethacrylate (PMMA), micronized TEFLON, boron nitride, acrylate copolymers, aluminum silicate, aluminum starch octenylsuccinate, bentonite, calcium silicate, cellulose, chalk, corn starch, diatomaceous earth, fuller's earth, glyceryl starch, hectorite, hydrated silica, kaolin, magnesium aluminum silicate, magnesium trisilicate, maltodextrin, montmorillonite, microcrystalline cellulose, rice starch, silica, talc, mica, titanium dioxide, zinc laurate, zinc myristate, zinc rosinate, alumina, attapulgite, calcium carbonate, calcium silicate, dextran, nylon, silica silylate, silk powder, sericite, soy flour, tin oxide, titanium hydroxide, trimagnesium phosphate, walnut shell powder, and mixtures thereof.

Suitable lakes include, without limitation, those of red dyes from the monoazo, disazo, fluoran, xanthene, or indigoid families, such as Red 4, 6, 7, 17, 21, 22, 27, 28, 30, 31, 33, 34, 36, and Red 40; lakes of yellow pyrazole, monoazo, fluoran, xanthene, quinoline, dyes or salt thereof, such as Yellow 5, 6, 7, 8, 10, and 11; lakes of violet dyes including those from the anthroquinone family, such as Violet 2 as well as lakes of orange dyes, including Orange 4, 5, 10, 11, and the like.

The organic pigments may be surface treated. In certain embodiments, the organic pigments are surface treated. In certain embodiments, more than 1% of the surface of the organic pigment is surface treated. For example, the organic pigment may have between 1% and 50% (e.g., between 1% and 20%, between 1% and 10%) of its surface treated.

The compositions may comprise a $TiO_2$ pigment grind component (i.e. all of the $TiO_2$ present in the composition) that is more than 50%, more than 60%, more than 70%, more than 80%, more than 90%, or more than 95% rutile $TiO_2$ by weight of the $TiO_2$ component. In some embodiments, the $TiO_2$ component consists of rutile-$TiO_2$. In some embodiments, $TiO_2$ may be present in an amount greater than 0.01% by weight of the composition (e.g., 0.1% to 10% by weight of the composition, 0.05% to 10% by weight of the composition). In some embodiments, the $TiO_2$ in the pigment grind has a particle size greater than 200 nm. As used herein, particle size measurements may be performed with dynamic light scattering.

The ratio of $TiO_2$ and organic pigment may be altered to enhance the stability of the color in compositions dependent on the exact mixture and weight percentage of the pigment component (e.g., organic pigments, pigmentary $TiO_2$, and inorganic pigments). In some embodiments, the weight ratio of organic pigments (e.g., Red 7, Red 6, Red 27, Red 33, Blue 1, Yellow 5, Red 7 lake, Red 6 lake, Red 27 lake, Blue 1 lake, Yellow 5 lake) to pigmentary rutile $TiO_2$ is between 20:1 and 1:20 by weight. In certain embodiments, the weight ratio of organic pigments to rutile $TiO_2$ may be between 20:1 and 1:1 by weight (e.g., 20:1 to 10:1, 10:1 to 1:1, 8:1 to 2:1, 9:1 to 1:1, 8:1 to 1:1, 7:1 to 1:1, 6:1 to 1:1, 5:1 to 1:1, 4:1 to 1:1, 3:1 to 1:1, 2:1 to 1:1). In other embodiments, the weight ratio of organic pigments to rutile $TiO_2$ may be between 1:1 and 1:20 by weight (e.g., 1:1 to 1:10, 1:10 to 1:20, 1:5 to 1:15, 1:8 to 1:2, 1:9 to 1:1, 1:8 to 1:1, 1:7 to 1:1, 1:6 to 1:1, 1:5 to 1:1, 1:4 to 1:1, 1:3 to 1:1, 1:2 to 1:1).

Without wishing to be bound by theory, it is believed that the incorporation of metal oxide particles may enhance the color stability of the pigmented compositions. Furthermore, metal oxide particles with particle sizes of less than 100 μm or less than 50 μm or less than 25 μm or less than 10 μm or less than 5 μm or less than 1 μ or less than 500 nm result in compositions result in more homogenous color distributions in the bulk as compared to metal oxide particles with larger particles sizes. The metal oxide particles are selected from metal oxide particle is silica, alumina, zirconia, zinc oxide, indium tin oxide, ceria, and mixtures thereof, and/or wherein the said metal oxide particles are fumed metal oxide particles.

In certain embodiments, the metal oxide component comprises less than 10% or less than 5% or less than 1% or less than 0.1% titanium oxide by weight of the metal oxide component. In some embodiments, the composition does not comprise titanium oxide. In some embodiments, the composition may be characterized as having a reduced color shift after 8 hours of heating between 80°C and 100°C as compared to an otherwise identical composition without the attenuation grade $TiO_2$. For example, the compositions may comprise an amount of attenuation grade $TiO_2$ such that the composition may be characterized as having a $\Delta E$ of less than 5 after eight hours of heating at more than 80°C (e.g., between 80°C and 100°C).

The compositions may comprise additional pigments or particulate materials for ultraviolet light absorption or scattering such as zinc oxide particulates, or for other aesthetic characteristics such as pearlescence (e.g., mica, bismuth oxychloride). Exemplary inorganic pigments include, but are not limited to, inorganic oxides and hydroxides such as magnesium oxide, magnesium hydroxide, calcium oxide, calcium hydroxides, aluminum oxide, aluminum hydroxide, iron oxides ($\alpha$-$Fe_2O_3$, $\gamma$-$Fe_2O_3$, $Fe_3O_4$, FeO) and iron hydroxides including red iron oxide, yellow iron oxide and black iron oxide, titanium lower oxides, zirconium oxides, chromium oxides, chromium hydroxides, manganese oxides, manganese hydroxides, cobalt oxides, cobalt hydroxides, cerium oxides, cerium hydroxides, nickel oxides, nickel hydroxides, zinc oxides and zinc hydroxides and composite oxides and composite hydroxides such as iron titanate, cobalt titanate and cobalt aluminate and the like. Preferably, the inorganic oxide particles may be selected from silica, alumina, zinc oxide, and iron oxide particles, and mixtures thereof. In one embodiment, the inorganic pigments have a particle size from 5 nm to 500 microns, or from 5 nm to 250 microns, or from 10 nm to 100 microns. In some embodiments, the particle size (median) will be less than bout 5 microns or less than 1 micron. In some embodiments, the composition comprises less than 5% inorganic pigments (other than $TiO_2$) by weight of the composition. In some embodiments, the composition comprises less than 1% inorganic pigments (other than $TiO_2$) by weight of the composition. In some embodiments, the composition comprises less than 0.5% inorganic pigments (other than $TiO_2$) by weight of the composition. In some embodiments, the composition comprises less than 5% iron oxide by weight of the composition. In some embodiments, the composition comprises less than 1% iron oxide by weight of the composition. In some embodiments, the composition comprises less than 0.5% iron oxide by weight of the composition.

The total pigment content of the compositions (e.g., organic pigments, $TiO_2$ (e.g., pigmentary rutile $TiO_2$, inorganic pigments)) is typically less than 30% by weight of the composition (e.g., less than 20% by weight of the composition, less than 15% by weight of the composition, less than 12% by weight of the composition). In some embodiments, the total pigment content is between 5% and 15% by weight of the composition.

The pigmented composition typically has a mix of individual pigments to result in certain color shades of the composition. This pigment component may have decreased susceptibility to color shift. For example, in some embodiments, the composition does not have a (L*,a*,b*) color value with the specular component included ("SCI") of (L*=40±10, a*=54±20, b*=18±20). Persons of ordinary skill in the art are able to measure L*a*b* values, for example by the measurement procedure detailed in Example 1. In some embodiments, the composition has a change in color of less than 10 (e.g., less than 3, less than 2.5) after eight hours of heating at more than 80°C (e.g., between 80°C and 100°C, between 85°C and 95°C). Differences between points in color space can be calculated (or approximated) using standard Euclidean geometry of the color space. For example, the color difference may be calculated with equation (1):

$$\Delta E_{ab}^* = \sqrt{(L_2^* - L_1^*)^2 + (a_2^* - a_1^*)^2 + (b_2^* - b_1^*)^2} \qquad \text{(I)}$$

.

The pigments, and specifically the organic pigments, may be surface treated. In some embodiments, the $TiO_2$ is surface treated. In some embodiments, both the $TiO_2$ and the organic pigments are independently surface treated. In certain implementations, the $TiO_2$ (e.g., attenuation grade $TiO_2$, rutile $TiO_2$) and the organic pigment (e.g., Red 7, Red 6, Red 27, Red 33, Blue 1, Yellow 5, Red 7 lake, Red 6 lake, Red 27 lake, Blue 1 lake, Yellow 5 lake) are independently surface treated. In various embodiments, $TiO_2$, the organic pigment, and the inorganic pigment are independently surface treated. In certain implementations, the $TiO_2$ (e.g., attenuation grade $TiO_2$, rutile $TiO_2$) and the organic pigment (e.g., Red 7, Red 6, Red 27, Red 33, Blue 1, Yellow 5, Red 7 lake, Red 6 lake, Red 27 lake, Blue 1 lake, Yellow 5 lake) have the same surface treatment. In various embodiments, $TiO_2$, the organic pigment, and the inorganic pigment are independently surface treated. In some embodiments, $TiO_2$, the organic pigment, and the inorganic pigment each have the same surface treatment.

The surface treatment may be any such treatment that modifies the surface of the modifying agent and/or the first colorant. For example, the surface treatment may make the pigments more hydrophobic or more dispersible in a vehicle or may increase the adhesion of the pigments to a modifying agent. The surface of the pigments may, for example, be covalently or ionically bound to an organic molecule or silicon-based molecule or may be adsorbed thereto, or the pigments may be physically coated with a layer of material. The surface treatment compound may be attached to the pigment through any suitable coupling agent, linker group, or functional group (e.g., silane, ester, ether). The compound may comprise a hydrophobic portion which may be selected from, for example, alkyl, aryl, allyl, vinyl, alkylaryl, aryl-alkyl, organosilicone, di-organosilicone, dimethicones, methicones, polyurethanes, silicone-polyurethanes, and fluoro- or perfluoro-derivatives thereof. Other hydrophobic modifiers include lauroyl lysine, Isopropyl Titanium Triisostearate (ITT), ITT and Dimethicone (ITT/Dimethicone) cross-polymers, ITT and Amino Acid, ITT/Triethoxycaprylylsilane Crosspolymer, waxes (*e.g.,* carnauba), fatty acids (*e.g.,* stearates), HDI/Trimethylol Hexylactone Crosspolymer, PEG-8 Methyl Ether Triethoxysilane, aloe, jojoba ester, lecithin, Perfluoroalcohol Phosphate, and Magnesium Myristate (MM), to name a few. In some embodiments, the organic pigments and/or the $TiO_2$ (*e.g.,* pigmentary $TiO_2$, attenuation grade $TiO_2$, pigmentary $TiO_2$ and attenuation grade $TiO_2$) is surface treated. In specific embodiments, the organic pigments are ITT treated. In certain embodiments, the $TiO_2$ is ITT/Dimethicone treated. In various embodiments, the organic pigments and the $TiO_2$ are /Dimethicone treated. In specific embodiments, the organic pigments are /Dimethicone treated. In certain embodiments, the $TiO_2$ is ITT/Dimethicone treated. In various embodiments, the organic pigments and the $TiO_2$ are ITT treated. Specific surface treated organic pigments which may be used include (INCI names):

Blue 1 Lake (And) Isopropyl Titanium Triisostearate (And) Triethoxysilylethyl Polydimethylsiloxyethyl Dimethicone,

Red 6 Lake (And) Isopropyl Titanium Triisostearate (And) Triethoxysilylethyl Polydimethylsiloxyethyl Dimethicone,

Red 7 Lake (And) Isopropyl Titanium Triisostearate (And) Triethoxysilylethyl Polydimethylsiloxyethyl Dimethicone,

Red 30 Lake (And) Isopropyl Titanium Triisostearate (And) Triethoxysilylethyl Polydimethylsiloxyethyl Dimethicone,

Yellow 5 Lake (And) Isopropyl Titanium Triisostearate (And) Triethoxysilylethyl Polydimethylsiloxyethyl Dimethicone,

Yellow 6 Lake (And) Isopropyl Titanium Triisostearate (And) Triethoxysilylethyl Polydimethylsiloxyethyl Dimethicone,

and combinations thereof. Specific surface treated $TiO_2$ which may be used includes (INCI names):

Titanium Dioxide (*e.g.,* attenuated $TiO_2$, rutile $TiO_2$) (And) Isopropyl Titanium Triisostearate (And) Triethoxysilylethyl Polydimethylsiloxyethyl Dimethicone,

Titanium Dioxide (*e.g.,* attenuated $TiO_2$, rutile $TiO_2$) (And) Isopropyl Titanium Triisostearate (And) Alumina (And) Triethoxysilylethyl Polydimethylsiloxyethyl Dimethicone,

Titanium Dioxide (*e.g.,* attenuated $TiO_2$, rutile $TiO_2$) (And) Cyclopentasiloxane (And) PEG/PPG-18/18 Dimethicone (And) Isopropyl Titanium Triisostearate (And) Triethoxysilylethyl Polydimethylsiloxyethyl Dimethicone (And) Disteardimonium Hectorite (And) Tocopheryl Acetate,

and combinations thereof. Specific surface treated inorganic pigments which may be used include (INCI names):

Iron Oxides (CI 77491) (And) Isopropyl Titanium Triisostearate (And) Triethoxysilylethyl Polydimethylsiloxyethyl Dimethicone,
Iron Oxides (CI 77492) (And) Isopropyl Titanium Triisostearate (And) Triethoxysilylethyl Polydimethylsiloxyethyl Dimethicone,
Iron Oxides (CI 77499) (And) Isopropyl Titanium Triisostearate (And) Triethoxysilylethyl Polydimethylsiloxyethyl Dimethicone,
Chromium Oxide Greens (And) Isopropyl Titanium Triisostearate (And) Triethoxysilylethyl Polydimethylsiloxyethyl Dimethicone,
Ferric Ammonium Ferrocyanide (And) Isopropyl Titanium Triisostearate (And) Triethoxysilylethyl Polydimethylsiloxyethyl Dimethicone,
Iron Oxides (CI 77491) (And) Cyclopentasiloxane (And) PEG/PPG-18/18 Dimethicone (And) Isopropyl Titanium Triisostearate (And) Triethoxysilylethyl Polydimethylsiloxyethyl Dimethicone (And) Disteardimonium Hectorite (And) Tocopheryl Acetate,
Iron Oxides (CI 77492) (And) Cyclopentasiloxane (And) PEG/PPG-18/18 Dimethicone (And) Isopropyl Titanium Triisostearate (And) Triethoxysilylethyl Polydimethylsiloxyethyl Dimethicone (And) Disteardimonium Hectorite (And) Tocopheryl Acetate,
Iron Oxides (CI 77499) (And) Cyclopentasiloxane (And) PEG/PPG-18/18 Dimethicone (And) Isopropyl Titanium Triisostearate (And) Triethoxysilylethyl Polydimethylsiloxyethyl Dimethicone (And) Disteardimonium Hectorite (And) Tocopheryl Acetate,
and combinations thereof. Surface treated components are available from KOBO products (New Jersey, USA) such as those listed as TTB or ITT products.

The surface treatment may comprise, in some embodiments, a material selected from aluminum laurate, aluminum stearate, an amino acid, chitin, collagen, fluorochemical, lecithin, metal soap, natural wax, polyacrylate, polyethylene, silicone, silane, titanatate ester, urethane, dimethicone, perfluoropolymethylisopropyl ether, styrene acrylates copolymer, magnesium myristate, lauroyl lysine and a combination thereof. In other embodiments, the surface treatment comprises a material selected from methicone, triethoxycaprylylsilane, trimethoxycaprylylsilane, dimethicone copolyol and a combination thereof.

The compositions may be in the form of an emulsion. Typically, the emulsions may comprise water and/or glycerin. The emulsions may be a water-in-oil, oil-in-water, silicone-in-water, water-in-silicone, polyol-in-oil, oil-in-polyol, glycerin-in-oil, oil-in-glycerin, silicone-in-glycerin, glycerin-in-silicone, silicone-in-polyol, or polyol-in-silicone emulsion. In preferred embodiments, the emulsion is a water-in-oil, oil-in-water, silicone-in-water, or water-in-silicone emulsion. The emulsions may comprise a non-aqueous external phase (*e.g.,* oil phase, silicone phase). In some embodiments, the emulsions may comprise an aqueous, a polyol, or a glycerin internal phase. In some embodiments the composition may comprise from 1-40% (*e.g.,* 1% to 10%, 20% to 30%, 30% to 40%) of the internal phase (*e.g.,* glycerin)

In some embodiments, the external (continuous) phase is an emollient oil phase. For example, the continuous oil phase may comprise any suitable oils for emulsions, including, without limitation, vegetable oils; fatty acid esters; fatty alcohols; isoparaffins such as isododecane and isoeicosane; hydrocarbon oils such as mineral oil, petrolatum, and polyisobutene; polyolefins and hydrogenated analogs thereof (*e.g.,* hydrogenate polyisobutene); natural or synthetic waxes; silicone oils such as dimethicones, cyclic silicones, and polysiloxanes; and the like. In certain implementations, the composition comprises from external phase carrier comprises from 20% to 50% emollient (*e.g.,* silicone such as diphenyl dimethicone, ester oil such as ethylhexyl palmitate) by weight of the composition.

Suitable ester oils include fatty acid esters. Special mention may be made of those esters commonly used as emollients in cosmetic formulations. Such esters will typically be the etherification product of an acid of the form $R_4$ $(COOH)_{1-2}$ with an alcohol of the form $R_5(OH)_{1-3}$ where $R_4$ and $R_5$ are each independently linear, branched, or cyclic hydrocarbon groups, optionally containing unsaturated bonds (*e.g.,* from 1-6 or 1-3 or 1), and having from 1 to 30 (*e.g.,* 6-30 or 8-30, or 12-30, or 16-30) carbon atoms, optionally substituted with one or more functionalities including hydroxyl, oxa, oxo, and the like. Preferably, at least one of $R_4$ and $R_5$ comprises at least 8, or at least 10, or at least 12, or at least 16 or at least 18 carbon atoms, such that the ester comprises at least one fatty chain. The esters defined above will include, without limitation, the esters of mono-acids with mono-alcohols, mono-acids with diols and triols, diacids with mono-alcohols, and tri-acids with mono-alcohols.

Suitable fatty acid esters include, without limitation, butyl acetate, butyl isostearate, butyl oleate, butyl octyl oleate, cetyl palmitate, ceyl octanoate, cetyl laurate, cetyl lactate, cetyl isononanoate, cetyl stearate, diisostearyl fumarate, diisostearyl malate, neopentyl glycol dioctanoate, dibutyl sebacate, di-$C_{12-13}$ alkyl malate, dicetearyl dimer dilinoleate, dicetyl adipate, diisocetyl adipate, diisononyl adipate, diisopropyl dimerate, triisostearyl trilinoleate, octodecyl stearoyl stearate, hexyl laurate, hexadecyl isostearate, hexydecyl laurate, hexyldecyl octanoate, hexyldecyl oleate, hexyldecyl palmitate, hexyldecyl stearate, isononyl isononanaote, isostearyl isononate, isohexyl neopentanoate, isohexadecyl stearate, isopropyl isostearate, n-propyl myristate, isopropyl myristate, n-propyl palmitate, isopropyl palmitate, hexacosanyl palmitate, lauryl lactate, octacosanyl palmitate, propylene glycol monolaurate, triacontanyl palmitate, dotriacontanyl palmitate, tetratriacontanyl palmitate, ethylhexyl palmitate, hexacosanyl stearate, octacosanyl stearate, triacontanyl stearate, dotriacontanyl stearate, stearyl lactate, stearyl octanoate, stearyl heptanoate, stearyl stearate, tetratriacontanyl stearate, triarachidin, tributyl citrate, triisostearyl citrate, tri-$C_{12-13}$-alkyl citrate, tricaprylin, tricaprylyl citrate, tridecyl behenate, trioctyldodecyl citrate, tridecyl cocoate, tridecyl isononanoate, glyceryl monoricinoleate, 2-octyldecyl palmitate, 2-octyldodecyl myristate or lactate, di(2-ethylhexyl)succinate, tocopheryl acetate, and the like.

Other suitable esters include those wherein $R_5$ comprises a polyglycol of the form H-(O-CHR*-CHR*)$_n$- wherein R* is independently selected from hydrogen or straight chain $C_{1-12}$ alkyl, including methyl and ethyl, as exemplified by polyethylene glycol monolaurate.

The oil may also comprise a volatile or non-volatile silicone oil. Suitable silicone oils include linear or cyclic silicones such as polyalkyl- or polyarylsiloxanes, optionally comprising alkyl or alkoxy groups having from 1 to 10 carbon atoms. Representative silicone oils include, for example, caprylyl methicone, cyclomethicone, cyclopentasiloxane decamethylcyclopentasiloxane, decamethyltetrasiloxane, diphenyl dimethicone, dodecamethylcyclohexasiloxane, dodecamethylpentasiloxane, heptamethylhexyltrisiloxane, heptamethyloctyltrisiloxane, hexamethyldisiloxane, methicone, methyl-phenyl polysiloxane, octamethylcyclotetrasiloxane, octamethyltrisiloxane, perfluorononyl dimethicone, polydimethylsiloxanes, and combinations thereof. The silicone oil will typically, but not necessarily, have a viscosity of between 5 and 3,000 centistokes (cSt), preferably between 50 and 1,000 cSt measured at 25° C.

In one embodiment, the silicone oil comprises phenyl groups, as is the case for a silicone oil such as methylphenylpolysiloxane (INCI name diphenyl dimethicone), commercially available from Shin Etsu Chemical Co under the name including F-5W, KF-54 and KF-56. Diphenyl dimethicones have good organic compatibility and may impart film-forming characteristics to the product. Further, the presence of phenyl groups increases the refractive index of the silicone oil and thus may contribute to high gloss of product if desired. In one embodiment, the silicone oil will have a refractive index of at least 1.3, preferably at least 1.4, more preferably at least 1.45, and more preferred still at least 1.5, when measured at 25° C. Another suitable phenyl-functionalized silicone oil has the INCI name phenyltrimethicone and is sold under the trade name DC 556 by Dow Corning. DC 556 has a refractive index of 1.46. In one embodiment, the silicone oil is a fluorinated silicone, such as a perfluorinated silicone (i.e., fluorosilicones). Fluorosilicones are advantageously both hydrophobic and oleophobic and thus contribute to a desirable slip and feel of the product. Fluorosilicones also impart long-wearing characteristics to a lip product. Fluorosilicones can be gelled with behenyl behenate if desired. One suitable fluorosilicone is a fluorinated organofunctional silicone fluid having the INCI name perfluorononyl dimethicone. Perfluorononyl dimethicone is commercially available from Pheonix Chemical under the trade name PECOSIL®. The compositions may comprise between 5% and 75% emollient (e.g., silicone oil, ester oil) by weight of the composition (e.g., between 10% and 60% by weight of the composition, between 20% and 50% by weight of the composition, between 25% and 45% by weight of the composition).

The compositions may also comprise hydrocarbon oils. Exemplary hydrocarbon oils are straight or branched chain paraffinic hydrocarbons having from 5 to 80 carbon atoms, typically from 8 to 40 carbon atoms, and more typically from 10 to 16 carbon atoms, including but not limited to, pentane, hexane, heptane, octane, nonane, decane, undecane, dodecane, tetradecane, tridecane, and the like. Some useful hydrocarbon oils are highly branched aliphatic hydrocarbons, including $C_{8-9}$ isoparaffins, $C_{9-11}$ isoparaffins, $C_{12}$ isoparaffin, $C_{20-40}$ isoparaffins and the like. Special mention may be made of the isoparaffins having the INCI names isohexadecane, isoeicosane, and isododecane (IDD).

Also, suitable as hydrocarbon oils are poly-$\alpha$-olefins, typically having greater than 20 carbon atoms, including (optionally hydrogenated) $C_{24-28}$ olefins, $C_{30-45}$ olefins, polyisobutene, hydrogenated polyisobutene, hydrogenated polydecene, polybutene, hydrogenated polycyclopentane, mineral oil, pentahydrosqualene, squalene, squalane, and the like. The hydrocarbon oil may also comprise higher fatty alcohols, such as oleyl alcohol, octyldodecanol, and the like.

Other suitable oils include without limitation castor oil, $C_{10-18}$ triglycerides, caprylic/capric/triglycerides, coconut oil, corn oil, cottonseed oil, linseed oil, mink oil, olive oil, palm oil, illipe butter, rapeseed oil, soybean oil, sunflower seed oil, walnut oil, avocado oil, camellia oil, macadamia nut oil, turtle oil, mink oil, soybean oil, grape seed oil, sesame oil, maize oil, rapeseed oil, sunflower oil, cottonseed oil, jojoba oil, peanut oil, olive oil, and combinations thereof.

Any one of the foregoing ester oils, silicone oils, and hydrocarbon oils are contemplated to be useful in the compositions described herein. Accordingly, in one embodiment, the compositions comprise at least one oil selected from the ester oils, silicone oils, and hydrocarbon oils described above. In another embodiment, the compositions comprise two or more oils selected from the ester oils, silicone oils, and hydrocarbon oils described above. In yet another embodiment, the compositions will comprise at least one ester, at least one silicone oil, and at least one hydrocarbon oil from the list above. Because the ester oils described herein function as emollients, it may be advantageous for the compositions comprise at least one ester oil, and may optionally comprise at least one additional oil selected from hydrocarbon oils, silicone oils, and combinations thereof.

Although not required, the compositions typically have an aqueous phase. In most embodiments, the aqueous phase is the discontinuous phase of emulsions (e.g., water-in-oil, glycerin-in-oil). As hydrolysis of the organic pigments may cause the color shift, these compositions are most affected by the increased color stability provided by the compositions of the invention. In some embodiments, the aqueous phase solvents are present from 1% to 50% (e.g., 1% to 20%, 5 to 15%, 1 to 10%) by weight of the composition. In other embodiments, the compositions are anhydrous.

Waxes and/or fillers may also be optionally added (particularly in those embodiments where the composition is a free standing solid at room temperature), in an amount ranging from at or 1% to or including 20% by weight of the composition or ranging from at or 1% to or including 10% by weight of the composition. Examples of fillers may include, but are not limited to silica, PMMA, nylon, alumina, barium sulfate, or any other filler used in such compositions. Examples of waxes may include, but are not limited to, linear polyethylene, microcrystalline petroleum wax, carnauba wax, lignite wax, ouricouri wax, rice bran wax, castor wax, mortar wax, stearone, acrawax, bayberry wax, castor wax, Japan wax, ozokerite, beeswax, candelilla wax, petrolatum, ceresin wax, cocoa buter, illipe butter, esparto wax, shellac wax, ethylene glycol diesters or triesters of $C_{18}$-$C_{36}$ fatty acids, cetyl palmitate, hard tallow, paraffin wax, lanolin, lanolin alcohol, cetyl alcohol, glyceryl monostearate, sugarcane wax, jojoba wax, stearyl alcohol, silicone waxes, and combinations thereof.

In some embodiments, the oil phase can include one or more waxes. Waxes may impart body to the emulsion so that the emulsion has the physical form of a semi-solid or solid. As used herein, the term "solid" is intended to refer to a composition that is self-supporting and capable of being molded into a free-standing stick (e.g., a lip stick). In some embodiments, the waxes are present in an amount sufficient to make the emulsion a solid emulsion. For example, the solid emulsion can have a hardness of at least 30 g. The composition typically has hardness at room temperature of at least 40 g. In one embodiment, the composition may have a substantially greater hardness, between 100 and 300 g. The hardness of an emulsion may be measured on a Texture Analyzer Model QTS-25 equipped with a 4 mm stainless steel probe (TA-24), as described in Avon's U.S. Pat. No. 8,580,283.

The waxes may be natural, mineral and/or synthetic waxes. Natural waxes include those of animal origin (e.g.,

beeswax, spermaceti, lanolin, and shellac wax) and those of vegetable origin (*e.g.,* carnauba, candelilla, bayberry, and sugarcane wax). Mineral waxes include, without limitation ozokerite, ceresin, montan, paraffin, microcrystalline, petroleum, and petrolatum waxes. Synthetic waxes include, for example, polyethylene glycols such as PEG-18, PEG-20, PEG-32, PEG-75, PEG-90, PEG-100, and PEG-180 which are sold under the tradename CARBOWAX® (The Dow Chemical Company). Mention may be made of the polyethylene glycol wax CARBOWAX 1000 which has a molecular weight range of 950 to 1,050 and a melting point of 38° C., CARBOWAX 1450 which has a molecular weight range of 1,305 to 1,595 and a melting point of 56° C., CARBOWAX 3350 which has a molecular weight range of 3,015 to 3,685 and a melting point of 56° C., and CARBOWAX 8000 which has a molecular weight range of 7,000 to 9,000 and a melting point of 61° C.

Synthetic waxes also include Fischer Tropsch (FT) waxes and polyolefin waxes, such as ethylene homopolymers, ethylene-propylene copolymers, and ethylene-hexene copolymers. Representative ethylene homopolymer waxes are commercially available under the tradename POLYWAX® Polyethylene (Baker Hughes Incorporated) with melting points ranging from 80° C. to 132° C. Commercially available ethylene-$\alpha$-olefin copolymer waxes include those sold under the tradename PETROLITE® Copolymers (Baker Hughes Incorporated) with melting points ranging from 95° C. to 115° C.

In one embodiment, the emulsion includes, in the oil phase, at least one wax selected from arcawax (N,N'-ethylenebisstearamide), microcrystalline wax, linear polyethylene wax, stearone (18-pentatriacontanone), castor wax, montan wax, lignite wax, ouricouri wax, carnauba wax, rice bran wax, shellac wax, esparto wax, ozokerite wax, jojoba wax, candelilla wax, ceresin wax, beeswax, castor wax, sugarcane wax, stearyl alcohol, hard tallow, cetyl alcohol, petrolatum, glyceryl monostearate, Japan wax, silicone wax, paraffin wax, lanolin wax, lanolin alcohol, bayberry wax, cetyl palmitate, illipe butter, cocoa butter, and ethylene glycol di- or tri-esters of $C_{18-36}$ fatty acids.

The amount of wax, if present, may be less than 2% (*e.g.,* 0.1-2%) by weight of the composition if the composition is a liquid or if clarity is desired. The amount of wax, if present, will typically be greater than 10% (*e.g.,* 10-20%) by weight of the composition if the composition is a semisolid or solid or if clarity is not a concern. In some embodiments, the emulsion may comprise wax from 1% to 25% (or 1-20% or 1-5% or 1-10%) by weight of the composition, particularly in embodiments formulated as lip sticks.

In one embodiment, composition includes, in the oil phase, from 0.1-2% or 2-5% or 5-10% or 10-15% or 15-20% by weight of at least one wax (*e.g.,* microcrystalline wax, ozokerite wax, polyethylene wax, paraffin wax, petrolatum wax). In one embodiment, the composition includes, in the oil phase, microcrystalline wax within the foregoing amounts. In one embodiment, the composition includes, in the oil phase, ozokerite wax within the foregoing amounts. In one embodiment, the composition includes, in the oil phase, polyethylene wax within the foregoing amounts. In one embodiment, the composition includes, in the oil phase, petrolatum wax within the foregoing amounts. In one embodiment, the composition includes, in the oil phase, paraffin wax within the foregoing amounts.

Typically, emulsions according to the invention further comprise one or more emulsifiers. For example, the one or more emulsifiers may be present in a total range from 0.01% to 10.0% by weight of the emulsion. In some embodiments, the total amount of emulsifier ranges from 0.1% to 6.0% be weight, or from 0.5% to 4.0% by weight of the emulsions. Examples of emulsifiers include polyglyceryl compounds such as polyglyceryl-6-polyricinoleate, polyglyceryl pentaoleate, polyglyceryl-isostearate, and polyglyceryl-2-diisostearate; glycerol esters such as glycerol monostearate or glycerol monooleate; phospholipids and phosphate esters such as lecithin and trilaureth-4-phosphate (available under the tradename HOSTAPHAT®KL-340-D); sorbitan-containing esters (including SPAN® esters) such as sorbitan laurate, sorbitan oleate, sorbitan stearate, or sorbitan sesquioleate; polyoxyethylene phenols such as polyoxyethylene octyl phenol; polyoxyethylene ethers such as polyoxyethylene cetyl ether and polyoxyethylene stearyl ether; polyethylene glycol emulsifiers such as PEG-30-polyhydroxystearate or alkylpolyethylene glycols; polypropylene glycol emulsifiers such as PPG-6-laureth-3; dimethicone polyols and polysiloxane emulsifiers; and the like. Combinations of emulsifiers, such as the combination of lecithin and sorbitan, are envisioned. Additional emulsifiers are provided in the INCI Ingredient Dictionary and Handbook, 12th Edition, 2008.

Additional components may be incorporated for various functional purposes as is customary in the cosmetic arts into the composition, and specifically the internal phase of emulsions, the external phase of emulsions, or as a particulate phase. However, while additional components consistent to formulate the above cosmetic compositions may be included, the inclusion of additional ingredients is limited to those ingredients in amounts which do not interfere with the formation or stability of the compositions (e.g., emulsions).

Such components may be selected from the group consisting of film-formers, pigments, waxes, emollients, moisturizers, preservatives, flavorants, antioxidants, botanicals, and mixtures thereof. Particular mention may be made of highly purified botanical extracts or synthetic agents which may have wound-healing, anti-inflammatory, or other benefits useful for treating the skin or lips. Additional embodiments may include antioxidants such as tocopherol and/or $\alpha$-hydroxy acids like glycolic acid, and lactic acid. The compositions may include one or more film-formers to increase the substantivity of the product.

Film formers, including film forming polymers, may also be employed. The term film-forming polymer may be understood to indicate a polymer which is capable, by itself or in the presence of at least one auxiliary film-forming agent, of forming a continuous film which adheres to a surface and functions as a binder for the particulate material. Polymeric film formers include, without limitation, acrylic polymers or co-polymers, (meth)acrylates, alkyl(meth)acrylates, polyolefins, polyvinyls, polacrylates, polyurethanes, silicones, polyamides, polyethers, polyesters, fluoropolymers, polyethers, polyacetates, polycarbonates, polyamides, polyimides, rubbers, epoxies, formaldehyde resins, organosiloxanes, dimethicones, amodimethicones, dimethiconols, methicones, silicone acrylates, polyurethane silicones copolymers, cellulosics, polysaccharides, polyquaterniums, and the like. Suitable film formers include those listed in the Cosmetic Ingredient Dictionary (INCI and Handbook, 12th Edition (2008).

The composition may comprise one or more preservatives or antimicrobial agents, such as methyl, ethyl, or propyl paraben, and so on, in amounts ranging from 0.0001-5 wt % by weight of the total composition. The compositions may have other ingredients such as one or more anesthetics, anti-allergenics, antifungals, anti-inflammatories, antimicrobials, antiseptics, chelating agents, emollients, emulsifiers, fragrances, humectants, lubricants, masking agents, medicaments, moisturizers, pH adjusters, preservatives, protectants, soothing agents, stabilizers, sunscreens, surfactants, thickeners, viscosifiers, vitamins, or any combinations thereof.

In one embodiment, the emulsions according to the invention are provided as products for application to the lips. Such lip products may include lip cream, lip balm, lip gloss, medicated lip treatment, lip moisturizer, lip cosmetic, lip sunscreen, and lip flavorant. In one embodiment, the lip product is a creamy, flowable lip product. In certain embodiments, products according to the invention may have the consistency of a semi-viscous liquid or paste. In other embodiments, the product is a lipstick.

When formulated as lip products, the emulsions according to the invention may be packaged in a re-closeable container. Such containers may include an enclosure or chamber charged with the emulsion formulated as a cosmetic composition and a cap removably attached to the container or reversibly configured on the container. In one embodiment, a cap may be attached to a squeezable enclosure (e.g., formed of a pliant plastic material) such that the cap can be removed from the orifice of the squeezable enclosure and replaced upon completion of dispensing of the composition. A cap may be attached to the body of a squeezable enclosure (e.g., by screw threads, a snap fit, or the like), to facilitate re-sealing the squeezable enclosure for storage between uses. In one embodiment, the cap is reversibly attached to the container for sealing the contents when in a closed position and for permitting the contents of the container to be dispensed when in an open position. Various containers are envisioned, including without limitation click pens, barrel dispensers, pumps, air-less pumps, pressurized packages, hand-squeezed containers, a cosmetic applicator, and the like.

In other embodiments, the emulsions may be in the form of skin care emulsions (e.g., lotions, creams, gels), color cosmetics, mascaras, eye shadows, lip color, lip liner, foundation, concealer, make up remover, sunscreen, deodorants, to name a few.

A sunscreen for protecting the skin from damaging ultra-violet rays may also be included in the embodied compositions. In one embodiment, sunscreens may include, without limitation, those with a broad range of UVB and UVA protection, such as octocrylene, avobenzone, octyl methoxycinnamate, octyl salicylate, oxybenzone, homosylate, benzophenone, camphor derivatives, zinc oxide, triazine complexes (e.g., Tinsorb, Univul), and titanium oxide. When present, the sunscreen may be in an amount ranging from at or 0.01% to or including 70% by weight of the composition.

Suitable exfoliating agents may include, for example α-hydroxy acids, β-hydroxy acids, oxaacids, oxadiacids, and their derivatives such as esters, anhydrides, and salts thereof. Suitable hydroxy acids include, for example, glycolic acid, lactic acid, malic acid, tartaric acid, citric acid, 2-hydrocxyalkanoic acid, mandelic acid, salicylic acid, and derivatives thereof. In some embodiments, the exfoliating agent is lactic acid. When present, the exfoliating agent may be in an amount ranging from at or 0.1% to or including 80% by weight of the composition.

The embodied compositions described here may further comprise one or more cosmetic powders or particulates, for example, calcium aluminum borosilicate, PMMA, polyethylene, polystyrene, methyl methacrylate crosspolymer, nylon-12, ethylene/acrylic acid copolymer, boron nitride, Teflon, silica, and the like. Typically, especially for makeup compositions, the compositions described here may additionally include colorants or pigments to impart a desired color or effect. Non-limiting examples may include inorganic pigments, organic pigments, and lakes. Exemplary inorganic pigments may include, but are not limited to, metal oxides and metal hydroxides such as magnesium oxide, magnesium hydroxide, calcium oxide, calcium hydroxides, aluminum oxide, aluminum hydroxide, iron oxides ($\alpha$-$Fe_2O_3$, $\gamma$-$Fe_2O_3$, $Fe_3O_4$, $FeO$), red iron oxide, yellow iron oxide, black iron oxide, iron hydroxides, titanium dioxide, titanium lower oxides, zirconium oxides, chromium oxides, chromium hydroxides, manganese oxides, cobalt oxides, cerium oxides, diatomaceous earth, nickel oxides, zinc oxides, composite oxides, and composite hydroxides such as iron titanate, cobalt titanate, and cobalt aluminate. Non-metal oxides are also contemplated to be suitable including alumina and silica, ultramarine blue, Prussian blue, manganese violet, bismuth oxychloride, talc, mica, sericite, magnesium carbonate, calcium carbonate, magnesium silicate, aluminum magnesium silicate, silicate, titanated mica, iron oxide titanated mica, bismuth oxychloride, and the like. Organic pigments may include, but are not limited to, at least one of carbon black, carmine, phthalocyanine blue and green pigment, diarylide yellow and orange pigments, and azo-type red and yellow pigments such as toluidine red, litho red, naphthol red and brown pigments, and combinations thereof.

In some embodiments, the compositions comprise polysaccharide thickener. In some embodiments, the compositions comprise an anionic polysaccharide (*e.g.,* xanthan gum). In other embodiments, the compositions do not comprise a polysaccharide thickener or the composition comprises less than 5%, less than 1%, or less than 0.1% a polysaccharide thickener by weight of the composition.

In one embodiment, the topical compositions (or aqueous phase thereof including an internal glycerin phase) may have a pH ranging from 1 to 7, but typically have a pH ranging from 3 to 6. In some embodiments, the internal phase has a pH between 3 and 5 or between 3.5 and 4.5 or between 3.7 and 3.9. Suitable pH adjusters such as, but not limited to, sodium hydroxide, citric acid and triethanolamine may be included in the described composition to bring the pH within the desired range.

Exemplary ranges of composition ingredients are provided in Table 1 (percentages are listed as weight percentage of the composition) for a cosmetic composition, such as a lipstick. It should be noted that some components are optional. Additionally, the Pigment Component may comprise organic pigments, $TiO_2$ (*e.g.,* anatase $TiO_2$, rutile $TiO_2$, a $TiO_2$ component consisting predominantly of rutile $TiO_2$) and additional inorganic pigments (optional) in the specified amounts. In some embodiments, the compositions comprise an emulsifier, a fragrance oil, a powder, a preservative, a sweetening agent, a sunscreen agent, or combinations thereof.

Table 1

| Component | Weight Percent (%) |
|---|---|
| Emollient | 1-60% |
| Emulsifier | 0.1-10% |
| Glycerin (Humectant) | 1-40% |
| Additional Ingredients | 0-0.1-20% |
| Wax | 2-30% |
| Water | 1-10% |
| Pigment Component | 1-30% |
|    -Organic Pigments | 0.5-20% |
|    -pigmentary $TiO_2$ | 0.5-20% |
|    -Inorganic Pigments | 0.01-10% |
| Metal oxide particle (< 50 $\mu$m particle size) such as fumed alumina | 0.1%-15% |

Compositions may be formulated by preparing a pigment grind, wherein inorganic pigments, organic pigments, and pigmentary $TiO_2$ may be dispersed in a suitable carrier via shear. The pressure and/or shear (*e.g.,* rubbing, brushing, combing) that is applied to the pigment grind may be provided by any suitable means for dispersing the pigment in the carrier, for example, by a three-roll mill. In some embodiments, each of the pigments are surface treated. Suitable carriers include ester oils such as ethylhexyl palmitate and silicones such as diphenyl dimethicone. In certain embodiments, the pigment grind may then be used formulated as an emulsion.

Table 2 shows several Pigment Grind Formulations which may be incorporated into the pigmented compositions described herein.

Table 2

| Component | Weight Percent in Pigment Grind |
|---|---|
| Grind Formulation 1 | |
| $TiO_2$ (Untreated or ITT Treated) | 7.00% |
| Red 7 (ITT Treated) | 8.40% |
| Red 6 (ITT Treated) | 34.53% |
| Black Iron Oxide (ITT Treated) | 0.07% |
| Diphenyl Dimethicone | 50% |
| Grind Formulation 2 | |
| $TiO_2$ (Untreated or ITT Treated) | 18.48% |

(continued)

| Grind Formulation 2 | |
|---|---|
| Red 7 (ITT Treated) | 27.13% |
| Blue 1 (ITT Treated) | 48.00% |
| Red 27 (ITT Treated) | 3.91% |
| Diphenyl Dimethicone | 50% |
| Grind Formulation 3 | |
| TiO$_2$ (Untreated or ITT Treated) | 13.96% |
| Red 7 (ITT Treated) | 32.97% |
| Red Iron Oxide (ITT Treated) | 0.55% |
| Blue 1 (ITT Treated) | 2.52% |
| Diphenyl Dimethicone | 50% |
| Grind Formulation 4 | |
| Rutile TiO$_2$ (ITT Treated) | 32.50% |
| Cosmetic Red Oxide (ITT Treated) | 8.20% |
| Yellow 5 Lake (ITT Treated) | 4.00% |
| Red Iron Oxide (ITT Treated) | 5.30% |
| Ethylhexyl Palmitate | 50% |
| Grind Formulation 5 | |
| Rutile TiO$_2$ (TTB Treated) | 32.50% |
| Cosmetic Red Oxide (TTB Treatment) | 12.20% |
| Yellow 5 Lake (TTB Treated) | 4.00% |
| Black Iron Oxide (TTB Treated) | 1.30% |
| Ethylhexyl Palmitate | 50% |
| Grind Formulation 6 | |
| Rutile TiO$_2$ (TTB Treated) | 35.00% |
| Red 7 (TTB Treated) | 10.00% |
| Yellow Iron Oxide (TTB Treated) | 5.00% |
| Ethylhexyl Palmitate | 50% |

Pigment grinds may also be used to construct an emulsion with a glycerin internal phase. In some embodiments, the emulsions may comprise from 5%-25% pigment grind by weight of the composition. An exemplary formulation is shown in Table 3. It will be understood that the pigment grind weight percentage indicated in Table 3 includes 50% diphenyl dimethicone. Accordingly, the composition illustrated in Table 3 comprises 55.11% emollients by weight of the composition.

Table 3

| Component | Weight Percent (%) |
|---|---|
| $\alpha$-Hydroxy Acid | 2.00 |
| Base | 0.35 |
| Emollient | 50.11 |
| Emulsifier | 3.75 |
| Fragrance Oil | 0.13 |

(continued)

| Component | Weight Percent (%) |
|---|---|
| Glycerin (Humectant) | 10.00 |
| Powder | 5.63 |
| Preservative | 0.50 |
| Sunscreen | 7.50 |
| Sweetener | 0.28 |
| Wax | 2.92 |
| Demineralized Water | 1.00 |
| Pigment Grind | 10.00 |

## EXAMPLES

The following examples illustrate specific aspects of the instant description. The examples should not be construed as limiting, as the example merely provides specific understanding and practice of the embodiments and its various aspects.

Comparative Example 1: Color Instability of Organic Pigment Compositions

Eight color formulations were prepared in both an anhydrous formulation and a glycerin-in-oil emulsion. For each color formulation, identical pigment grinds were placed in the anhydrous and the glycerin-in-oil emulsion at 10% by weight of the composition. The pigments in each Color Shade Formulation are shown in Table 4. Additionally, the L*a*b* space values ($\pm$ 5) with the specular component include ("SCI") for each Color Shade Formulation are shown in Table 4.

Table 4

| Color Shade Formulation | 1 | 2 | 3 | 4 | 5 | 6 | 7 | 8 |
|---|---|---|---|---|---|---|---|---|
| Titanium Dioxide (Anatase) | 1.91 | 11.42 | 7 | 18.48 | 37.06 | 0.5 | 13.96 | 22.41 |
| Red 7 Lake/ITT | 9.02 | 15.91 | 8.4 | 27.13 | 3.04 | 31.9 | 32.97 | 0 |
| Cosmetic Brown | 24.14 | 0 | 0 | 0 | 3.82 | 4.88 | 0 | 0 |
| Iron Oxide Red | 8.58 | 0 | 0 | 0 | 0 | 0 | 0 | 0 |
| Cosmetic Red Oxide | 6.35 | 13.04 | 0 | 0 | 0 | 4.67 | 0.55 | 0 |
| Fd&C Blue No.1 Aluminum Lake-ITT | 0 | 9.63 | 0 | 0.48 | 0 | 0 | 2.52 | 0 |
| D&C Red No.6 Barium Lake-ITT | 0 | 0 | 34.53 | 0 | 0 | 0 | 0 | 8.02 |
| Iron Oxide Black | 0 | 0 | 0.07 | 0 | 0 | 0.95 | 0 | 0 |
| D&C Red No.27 Aluminum Lake-ITT | 0 | 0 | 0 | 3.91 | 0 | 0 | 0 | 18.87 |
| Iron Oxide Red 34 | 0 | 0 | 0 | 0 | 5.47 | 0 | 0 | 0 |
| Iron Oxide-Yellow | 0 | 0 | 0 | 0 | 0.61 | 0 | 0 | 0.7 |
| Yellow 5 Lake-ITT | 0 | 0 | 0 | 0 | 0 | 7.1 | 0 | 0 |
| Initial L* | 19 | 17 | 33 | 25 | 37 | 18 | 22 | 40 |
| Initial a* | 26 | 16 | 53 | 38 | 19 | 32 | 25 | 54 |
| Initial b* | 24 | 9 | 43 | 1 | 12 | 25 | -3 | 18 |

The formulations were heated to 87°C. This temperature was chosen as an optimal hold temperature by evaluating the melt point of the lipstick base wax phase by Differential Scanning Calorimetry ("DSC") and determining a range where the full phase transition from solid to liquid of the wax phase of the tested formulations occurred. At hour intervals beginning when the emulsions reached 87°C (t=0), the change in color shade of each formulation from the initial heating time was measured ($\Delta E$). Table 4 shows the corresponding L*a*b* space coordinates of the initial color shade of each formulation at t=0.

To measure the color at each time point, the bulk is drawn over a Lanetta card with a consistent 3 mil thickness. Measurements were performed on the white portion of the Lanetta card. Spectrophotometric measurements were performed in the L*a*b* color space using a Konica Minolta CM-2600D spectrophotometer. This process was designed to reflect manufacturing stresses placed on the compositions thereby being indicative of the color shade stability and consistency of a formulation in mass production. Color does not typically shift upon cooling as the forces which accelerate shade instability are energy sources (e.g., heat, light). Table 4 shows the corresponding L*a*b* space coordinates of the initial color shade of each formulation at t=0.

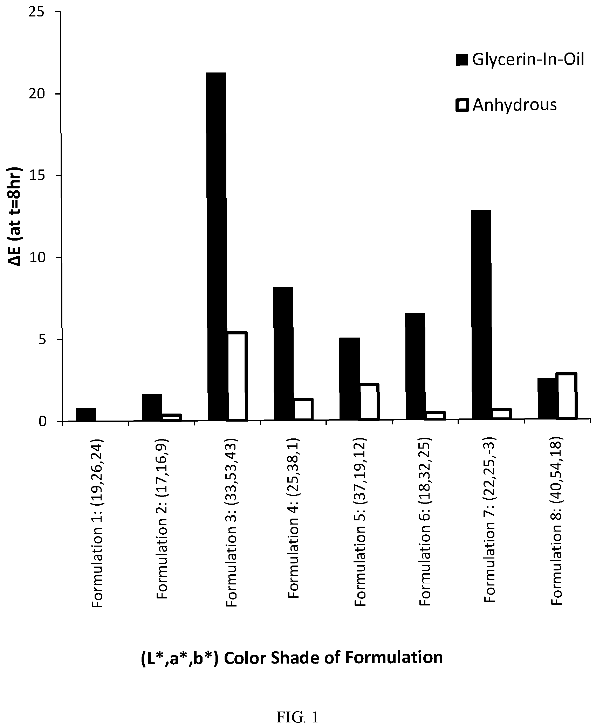

The change in color shade was monitored at hour intervals after each hour. At hour intervals beginning when the emulsions reached 87°C (t=0), the change in color shade of each formulation from the initial heating time was measured ($\Delta$E). FIG. 1 illustrates the change in color shade measurement for each of the anhydrous and glycerin-in-oil emulsion version of each Color Shade Formulation after eight hours of heating at 87°C ($\Delta$E between t=0 and t=8 hours).

As can be seen in FIG. 1, as between anhydrous and glycerin-in-oil emulsion versions of each Color Shade Formulation, the color shade stability in anhydrous formulations is generally much greater than in glycerin-in-oil emulsions across the L*a*b* coordinate system, with the exception of Color Shade 8 (L*=40, a*=54, b*=18).

As can also be seen in FIG. 1, some Color Shade Formulations, whether the anhydrous and glycerin-in-oil emulsion version, also are more affected by shifts in color shade than others (i.e., the instability also exists in anhydrous formulations, albeit to a lesser degree).

Example 2: Mitigating Color Shift In Formulations with ITT-treated Rutile Only TiO$_2$

Grind formulation 6 (shown in Table 2) was tested in various emulsions in order to demonstrate the effect the color shade stability afforded by the use of metal oxide particles with particle sizes of less than 100 $\mu$m or less than 50 $\mu$m. ITT Treated and ITT/dimethicone (TTB) treated compounds were purchased from KOBO products (New Jersey, USA).

Pigment Grind formulation 6 was formulated into glycerin-in-oil emulsions illustrated in Table 5.

Table 5

| Component | Control - Weight Percent (%) | Comparative TiO$_2$ Attenuation grade emulsion A - Weight Percent (%) | Comparative TiO$_2$ Attenuation grade emulsion B - Weight Percent (%) | Fumed Alumina emulsion - Weight Percent (%) | Comparative 100 $\mu$m Alumina emulsion - Weight Percent (%) |
|---|---|---|---|---|---|
| Emulsion internal phase formula | 29.89 | 29.89 | 29.89 | 29.89 | 29.89 |
| Emollient | 50.11 | 45.11 | 55.11 | 49.11 | 55.11 |
| Pigment Grind 6 | 20.00 | 20.00 | 10.00 | 20.00 | 10.00 |
| Fumed Alumina | 0.00 | 0.00 | 0.00 | 1.00 | 0.00 |
| Rutile TiO$_2$ attenuation grade (TTO-TTB7) | 0.00 | 5.00† | 5.00† | 0.00 | 0.00 |
| Alumina (100 $\mu$m) | 0.00 | 0.00 | 0.00 | 0.00 | 5.00 |
| Total Emollient | 50.11+10 = 60.11 | 45.11+10 = 55.11 | 45.11+5 = 50.11 | 49.11+10 = 59.11 | 55.11+5 = 60.11 |
| Initial L* | 46.47 | 41.47 | 39.61 | 37.54 | 40.74 |
| Initial a* | 34.08 | 29.07 | 29.91 | 33.79 | 33.72 |
| Initial b* | 6.18 | 5.80 | 9.59 | 4.71 | 3.52 |
| †0.55% - 0.925% of the formula corresponds to fumed alumina from the Rutile TiO$_2$ attenuation grade (TTO-TTB7) | | | | | |

Each emulsion was a glycerin-in-oil emulsion with an acidic internal phase (pH between 3.7 and 3.9). The initial L*a*b* space color values (SCI) were measured as described above in each batch with the initial L*a*b* color values shown in Table 5.

As can be seen, there is minimal color difference between the batches. The initial difference in color shade between control and the fumed alumina emulsion is, for example:

$$\Delta E = \sqrt{(37.54\text{-}46.47)^2+(33.79\text{-}34.08)^2+(4.71\text{-}6.18)^2} = 9.05$$

In these experiments, the emollients weight percent does not include the emollients added from the pigment grind (total emollients in each composition are indicated in Table 5). In these experiments, the attenuation grade $TiO_2$ was dispersed in the continuous phase rather than the pigment grind due to its high level of dispersibility.

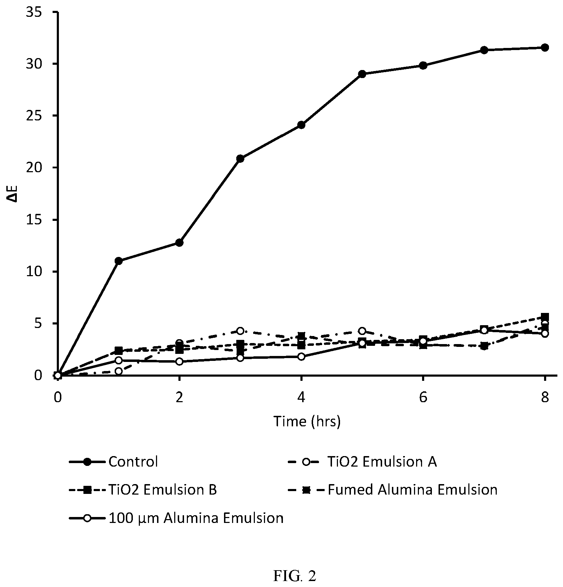

The emulsions were heated to 90°C. At hour intervals beginning when the emulsions reached 90°C (t=0), the change in color shade ($\Delta E$) for each emulsion from t=0 was measured as in Example 1. The $\Delta E$ values at each time point are shown in Table 6 and illustrated in FIG. 2.

Table 6

| Time (H) | Control | $TiO_2$ Emulsion A | $TiO_2$ Emulsion B | Fumed Alumina Emulsion | 100 $\mu$m Alumina Emulsion |
|---|---|---|---|---|---|
| 0 | 0 | 0.00 | 0.00 | 0.00 | 0.00 |
| 1 | 11.01 | 0.41 | 2.38 | 2.40 | 1.46 |
| 2 | 12.78 | 3.11 | 2.48 | 2.92 | 1.34 |
| 3 | 20.85 | 4.27 | 3.04 | 2.35 | 1.70 |
| 4 | 24.1 | 3.51 | 2.93 | 3.81 | 1.82 |
| 5 | 29.01 | 4.26 | 3.29 | 2.98 | 3.13 |
| 6 | 29.83 | 2.97 | 3.44 | 2.93 | 3.32 |
| 7 | 31.32 | 2.83 | 4.42 | 2.90 | 4.33 |
| 8 | 31.56 | 4.99 | 5.62 | 4.66 | 4.02 |

As can be seen, the control emulsion which did not contain any metal oxide particles had significant color instability. Those emulsions with a metal oxide particle (*e.g.,* $TiO_2$, alumina) were significantly more stable than the control emulsion. However, additional differences among the experimental formulations were also observed. The 100 $\mu$m alumina emulsion and $TiO_2$ emulsion B resulted in compositions with less uniform color density (an effect which was exacerbated at 8 hours for the 100 $\mu$m alumina emulsion). In each case, dark patches were produced in the bulk which would be problematic for mass manufacture of pigmented cosmetic compositions. Only the emulsion comprising metal oxide particles of less than 50 $\mu$m and a ratio of pigments (pigmentary $TiO_2$+organic pigments) to metal oxide of less than 1:1 (i.e. $TiO_2$ emulsion A and the fumed alumina emulsion) showed both homogenous color distribution and color stability. It should be noted that attempts to formulate $TiO_2$ emulsions like $TiO_2$ emulsion A but with less than 5% Rutile $TiO_2$ attenuation grade (TTO-TTB7) (*q.s.* with Emollient) resulted in a commercially unacceptable, unstable, yellowed product, whereas the fumed alumina emulsion is able to achieve similar stability with just 1% fumed alumina and none of the more costly rutile $TiO_2$ attenuation grade (TTO-TTB7).

**Claims**

**1.** A pigmented composition comprising:

(a) pigmentary titanium dioxide;
(b) organic pigments; and
(c) metal oxide particles having an average particle size of less than 50 $\mu$m (e.g., as measured by dynamic light scattering);

wherein the weight ratio of said pigmentary titanium dioxide and said organic pigments to said metal oxide particles in said composition is greater than 1:1 (e.g., 100:1 to 1:1, 50:1 to 1:1, 30:1 to 1:1, 100:1 to 2:1, 50:1 to 2:1, 30:1 to 2:1, 50:1 to 1:1, 100:1 to 5:1, 50:1 to 5:1, 30:1 to 5:1);

wherein said metal oxide particles are selected from silica, alumina, zirconia, zinc oxide, indium tin oxide, ceria, and mixtures thereof, and/or wherein said metal oxide particles are fumed metal oxide particles.

2. The pigmented composition according to claim 1, wherein said composition has a change in color ($\Delta$E) of less than 10 (e.g., of less than 5) after 8 hours at heating at more than 80 °C.

3. The pigmented composition according to claim 1 or 2, wherein said titanium dioxide consists predominantly of rutile phase titanium dioxide.

4. The pigmented composition according to any one of claims 1-3, wherein said metal oxide particles comprise fumed alumina (e.g., more than 70% or more than 80% or more than 90% or more than 95% or more than 99% of said metal oxide particles are fumed alumina by weight).

5. The pigmented composition according to any one of claims 1-4, wherein the weight ratio of said organic pigments to said pigmentary TiO$_2$ is between 20:1 and 1:20 (e.g., 20:1 to 10:1, 10:1 to 1:1, 8:1 to 2:1, 9:1 to 1:1, 8:1 to 1:1, 7:1 to 1:1, 6:1 to 1:1, 5:1 to 1:1, 4:1 to 1:1, 3:1 to 1:1, 2:1 to 1:1, 1:1 to 1:10, 1:10 to 1:20, 1:5 to 1:15, 1:8 to 1:2, 1:9 to 1:1, 1:8 to 1:1, 1:7 to 1:1, 1:6 to 1:1, 1:5 to 1:1, 1:4 to 1:1, 1:3 to 1:1, 1:2 to 1:1).

6. The pigmented composition according to any one of claims 1-5 further comprising water and/or glycerin, optionally wherein said composition is in the form of an emulsion and wherein said emulsion is a water-in-oil, oil-in-water, glycerin-in-oil, silicone-in-water, or water-in-silicone emulsion

7. The pigmented composition according to any one of claims 1-6, wherein said pigmentary titanium dioxide and organic pigments are treated with isopropyl triisostearyl titanate (ITT) or ITT/dimethicone.

8. The pigmented composition according to any one of claims 1-7, wherein said organic pigments comprise Red 7 and/or Red 6 and/or Red 27 and/or Blue 1 and/or Yellow 5 and/or Red 33, optionally wherein said organic pigments comprise between 5% and 40% Red 7 by weight of the composition.

9. The pigmented composition according to any one of claims 1-8, wherein said composition further comprises wax, optionally wherein said wax comprises paraffin wax, microcrystalline petroleum wax, or combinations thereof.

10. The pigmented composition according to any one of claims 1-9, wherein said composition is in the form of a solid stick, a liquid, a cream, a lotion, or a powder, optionally wherein said composition is in the form of a lipstick, lip color, mascara, eye liner, blush, bronzer, powder, eye shadow, nail polish, foundation, lotion, or concealer.

11. A pigmented composition in the form of a glycerin-in-oil emulsion comprising:

(a) from 0.1% to 10% rutile pigmentary TiO$_2$ by weight of the composition; and
(b) from 1% to 10% organic pigments (e.g.. Red 7, Red 6, Red 27, Red 33, Blue 1, Yellow 5) by weight of the composition;
(c) from 0.1% to 10% fumed alumina by weight of the composition;
(d) from 20% to 50% silicone by weight of the composition; and
(e) from 1% to 50% (e.g., 1% to 10%, 20% to 30%, 30% to 40%) glycerin by weight of the composition;

wherein the weight ratio of said pigmentary titanium dioxide and said organic pigments to said metal oxide particles in said composition is greater than 1:1, and wherein the metal oxide particles have a particle size of less than 50 $\mu$m.

12. The pigmented composition according to claim 11, wherein the weight ratio of said organic pigments to said rutile pigmentary TiO$_2$ is between 20:1 and 1:20.

13. The pigmented composition according to any one of claims 11-12, wherein the weight ratio of said rutile pigmentary titanium dioxide and said organic pigments to said fumed alumina in said composition is greater than 1:1 (e.g., 100:1 to 1:1, 50:1 to 1:1, 30: 1 to 1:1, 100:1 to 2:1, 50:1 to 2:1, 30:1 to 2:1, 50:1 to 1:1, 100:1 to 5:1, 50:1 to 5:1, 30:1 to 5:1).

14. A method for coloring a human integument comprising applying to said human integument a composition according to any one of claims 1-13.

**15.** The method according to claim 14, wherein said human integument is hair, skin, lips, or nails.

**Patentansprüche**

**1.** Pigmentierte Zusammensetzung, umfassend:

(a) pigmentiertes Titandioxid;
(b) organische Pigmente; und
(c) Metalloxidteilchen mit einer durchschnittlichen Teilchengröße von weniger als 50 μm (z. B. gemessen durch dynamische Lichtstreuung);

wobei das Gewichtsverhältnis des pigmentierten Titandioxids und der organischen Pigmente zu den Metalloxidteilchen in der Zusammensetzung größer als 1:1 ist (z.B. 100:1 zu 1:1, 50:1 zu 1:1, 30:1 zu 1:1, 100:1 zu 2:1, 50:1 zu 2:1, 30:1 zu 2:1, 50:1 zu 1:1, 100:1 zu 5:1, 50:1 zu 5:1, 30:1 zu 5:1); wobei die Metalloxidteilchen aus Siliziumdioxid, Aluminiumoxid, Zirkoniumdioxid, Zinkoxid, Indiumzinnoxid, Ceroxid und Mischungen davon ausgewählt sind, und/oder wobei die Metalloxidteilchen pyrogene Metalloxidteilchen sind.

**2.** Pigmentierte Zusammensetzung nach Anspruch 1, wobei die Zusammensetzung eine Farbänderung ($\Delta E$) von weniger als 10 (z. B. von weniger als 5) nach 8 Stunden Erhitzen auf mehr als 80 °C aufweist.

**3.** Pigmentierte Zusammensetzung nach Anspruch 1 oder 2, wobei das Titandioxid überwiegend aus Titandioxid in der Rutilphase besteht.

**4.** Pigmentierte Zusammensetzung nach einem der Ansprüche 1 bis 3, wobei die Metalloxidteilchen pyrogenes Aluminiumoxid umfassen (z. B. sind mehr als 70 % oder mehr als 80 % oder mehr als 90 % oder mehr als 95 % oder mehr als 99 % der Metalloxidteilchen pyrogenes Aluminiumoxid nach Gewicht).

**5.** Pigmentierte Zusammensetzung nach einem der Ansprüche 1-4, wobei das Gewichtsverhältnis der organischen Pigmente zu dem pigmentierten $TiO_2$ zwischen 20:1 und 1:20 liegt (z.B., 20:1 zu 10:1, 10:1 zu 1:1, 8:1 zu 2:1, 9:1 zu 1:1, 8:1 zu 1:1, 7:1 zu 1:1, 6:1 zu 1:1, 5:1 zu 1:1, 4:1 zu 1:1, 3:1 zu 1:1, 2:1 zu 1:1, 1:1 zu 1:10, 1:10 zu 1:20, 1:5 zu 1:15, 1:8 zu 1:2, 1:9 zu 1:1, 1:8 zu 1:1, 1:7 zu 1:1, 1:6 zu 1:1, 1:5 zu 1:1, 1:4 zu 1:1, 1:3 zu 1:1, 1:2 zu 1:1).

**6.** Pigmentierte Zusammensetzung nach einem der Ansprüche 1 bis 5; des Weiteren umfassend Wasser und/oder Glycerin, wobei die Zusammensetzung optional in Form einer Emulsion vorliegt und wobei die Emulsion eine Wasser-in-Öl-, Öl-in-Wasser-, Glycerin-in-Öl-, Silikon-in-Wasser- oder Wasser-in-Silikon-Emulsion ist

**7.** Pigmentierte Zusammensetzung nach einem der Ansprüche 1-6, wobei das pigmentierte Titandioxid und die organischen Pigmente mit Isopropyltriisostearyltitanat (ITT) oder ITT/Dimethicon behandelt sind.

**8.** Pigmentierte Zusammensetzung nach einem der Ansprüche 1-7, wobei die organischen Pigmente Red 7 und/oder Red 6 und/oder Red 27 und/oder Blue 1 und/oder Yellow 5 und/oder Red 33 umfassen, wobei die organischen Pigmente optional zwischen 5 und 40 Gew.-% Red 7 der Zusammensetzung umfassen.

**9.** Pigmentierte Zusammensetzung nach einem der Ansprüche 1 bis 8, wobei die Zusammensetzung des Weiteren Wachs enthält, wobei das Wachs optional Paraffinwachs, mikrokristallines Petroleumwachs oder Kombinationen davon umfasst.

**10.** Pigmentierte Zusammensetzung nach einem der Ansprüche 1 bis 9, wobei die Zusammensetzung in Form eines festen Stifts, einer Flüssigkeit, einer Creme, einer Lotion oder eines Puders vorliegt, wobei die Zusammensetzung optional in Form eines Lippenstifts, einer Lippenfarbe, von Mascara, eines Eyeliners, eines Rouges, eines Bronzers, eines Puders, eines Lidschattens, eines Nagellacks, einer Foundation, einer Lotion oder eines Concealers vorliegt.

**11.** Pigmentierte Zusammensetzung in Form einer Glycerin-in-Öl-Emulsion, umfassend:

(a) 0,1 % bis 10 % Rutilpigment TiO2, bezogen auf das Gewicht der Zusammensetzung; und
(b) 1 bis 10 % organische Pigmente (z. B. Red 7, Red 6, Red 27, Red 33, Blue 1, Yellow 5), bezogen auf das Gewicht der Zusammensetzung;

(c) 0,1% bis 10 % pyrogenes Aluminiumoxid, bezogen auf das Gewicht der Zusammensetzung;

(d) 20 % bis 50 % Silikon, bezogen auf das Gewicht der Zusammensetzung; und

(e) 1 % bis 50 % (z. B. 1 % bis 10 %, 20 % bis 30 %, 30 % bis 40 %) Glycerin, bezogen auf das Gewicht der Zusammensetzung;

wobei das Gewichtsverhältnis des pigmentierten Titandioxids und der organischen Pigmente zu den Metalloxidteilchen in der Zusammensetzung größer als 1:1 ist, und wobei die Metalloxidteilchen eine Teilchengröße von weniger als 50 μm aufweisen.

12. Pigmentierte Zusammensetzung nach Anspruch 11, wobei das Gewichtsverhältnis der organischen Pigmente zum Rutilpigment TiO₂ zwischen 20:1 und 1:20 liegt.

13. Pigmentierte Zusammensetzung nach einem der Ansprüche 11-12, wobei das Gewichtsverhältnis des Rutilpigment Titandioxid und der organischen Pigmente zu dem pyrogenen Aluminiumoxid in der Zusammensetzung größer ist als 1:1 (z.B., 100:1 zu 1:1, 50:1 zu 1:1, 30:1 zu 1:1, 100:1 zu 2:1, 50:1 zu 2:1, 30:1 zu 2:1, 50:1 zu 1:1, 100:1 zu 5:1, 50:1 zu 5:1, 30:1 zu 5:1).

14. Verfahren zum Färben eines menschlichen Integuments, umfassend das Auftragen einer Zusammensetzung nach einem der Ansprüche 1-13 auf das menschliche Integument.

15. Verfahren nach Anspruch 14, wobei es sich bei dem menschlichen Integument um Haare, Haut, Lippen oder Nägel handelt.

**Revendications**

1. Composition pigmentée comprenant :

(a) du dioxyde de titane pigmentaire ;
(b) des pigments organiques ; et
(c) des particules d'oxyde métallique présentant une taille de particule moyenne inférieure à 50 μm (par exemple, telle que mesurée par diffusion de lumière dynamique) ;

dans laquelle le rapport pondéral dudit dioxyde de titane pigmentaire et desdits pigments organiques auxdites particules d'oxyde métallique dans ladite composition est supérieur à 1:1 (par exemple, de 100:1 à 1:1, de 50:1 à 1:1, de 30:1 à 1:1, de 100:1 à 2:1, de 50:1 à 2:1, de 30:1 à 2:1, de 50:1 à 1:1, de 100:1 à 5:1, de 50:1 à 5:1, de 30:1 à 5:1) ;
dans laquelle lesdites particules d'oxyde métallique sont choisies parmi la silice, l'alumine, la zircone, l'oxyde de zinc, l'oxyde d'indium-étain, l'oxyde de cérium et des mélanges de ceux-ci, et/ou dans lequel lesdites particules d'oxyde métallique sont des particules d'oxyde métallique sublimé.

2. Composition pigmentée selon la revendication 1, dans laquelle ladite composition présente un changement de couleur (ΔE) inférieur à 10 (par exemple, inférieur à 5) après 8 heures de chauffage à plus de 80°C.

3. Composition pigmentée selon la revendication 1 ou 2, dans laquelle ledit dioxyde de titane est constitué principalement de dioxyde de titane en phase rutile.

4. Composition pigmentée selon l'une quelconque des revendications 1 à 3, dans laquelle lesdites particules d'oxyde métallique comprennent de l'alumine fumée (par exemple, plus de 70 % ou plus de 80 % ou plus de 90 % ou plus de 95 % ou plus de 99 % en poids desdites particules d'oxyde métallique sont de l'alumine fumée).

5. Composition pigmentée selon l'une quelconque des revendications 1 à 4, dans laquelle le rapport pondéral desdits pigments organiques audit TiO₂ pigmentaire est compris entre 20:1 et 1:20 (par exemple, de 20:1 à 10:1, de 10:1 à 1:1, de 8:1 à 2:1, de 9:1 à 1:1, de 8:1 à 1:1, de 7:1 à 1:1, de 6:1 à 1:1, de 5:1 à 1:1, de 4:1 à 1:1, de 3:1 à 1:1, de 2:1 à 1:1, de 1:1 à 1:10, de 1:10 à 1:20, de 1:5 à 1:15, de 1:8 à 1:2, de 1:9 à 1:1, de 1:8 à 1:1, de 1:7 à 1:1, de 1:6 à 1:1, de 1:5 à 1:1, de 1:4 à 1:1, de 1:3 à 1:1, de 1:2 à 1:1).

6. Composition pigmentée selon l'une quelconque des revendications 1 à 5, comprenant en outre de l'eau et/ou de la glycérine, facultativement dans laquelle ladite composition est sous la forme d'une émulsion et dans laquelle ladite émulsion est une émulsion eau-dans-huile, huile-dans-eau, glycérine-dans-huile, silicone-dans-eau ou eau-dans-silicone.

7. Composition pigmentée selon l'une quelconque des revendications 1 à 6, dans laquelle ledit dioxyde de titane pigmentaire et lesdits pigments organiques sont traités avec du titanate d'isopropyle et de triisostéaryle (ITT) ou de l'ITT/diméthicone.

8. Composition pigmentée selon l'une quelconque des revendications 1 à 7, dans laquelle lesdits pigments organiques comprennent du Red 7 et/ou du Red 6 et/ou du Red 27 et/ou du Blue 1 et/ou du Yellow 5 et/ou du Red 33, facultativement dans laquelle lesdits pigments organiques comprennent entre 5 % et 40 % de Red 7 en poids de la composition.

9. Composition pigmentée selon l'une quelconque des revendications 1 à 8, dans laquelle ladite composition comprend en outre de la cire, facultativement dans laquelle ladite cire comprend de la cire de paraffine, de la cire de pétrole microcristalline ou des combinaisons de celles-ci.

10. Composition pigmentée selon l'une quelconque des revendications 1 à 9, dans laquelle ladite composition est sous la forme d'un bâton solide, d'un liquide, d'une crème, d'une lotion ou d'une poudre, facultativement dans laquelle ladite composition est sous la forme d'un rouge à lèvres, d'une couleur pour les lèvres, d'un mascara, d'un eye-liner, d'un blush, d'une poudre bronzante, d'une poudre, d'un fard à paupières, d'un vernis à ongles, d'un fond de teint, d'une lotion ou d'un anti-cernes.

11. Composition pigmentée sous la forme d'une émulsion glycérine dans huile comprenant :

(a) de 0,1 % à 10 % de $TiO_2$ pigmentaire rutile en poids de la composition ; et
(b) de 1 % à 10 % de pigments organiques (par exemple, Red 7, Red 6, Red 27, Red 33, Blue 1, Yellow 5) en poids de la composition ;
(c) de 0,1 % à 10 % d'alumine sublimée en poids de la composition ;
(d) de 20 % à 50 % de silicone en poids de la composition ; et
(e) de 1 % à 50 % (par exemple, de 1 % à 10 %, de 20 % à 30 %, de 30 % à 40 %) de glycérine en poids de la composition ;

dans laquelle le rapport pondéral dudit dioxyde de titane pigmentaire et desdits pigments organiques auxdites particules d'oxyde métallique dans ladite composition est supérieur à 1:1, et dans lequel les particules d'oxyde métallique présentent une taille de particule inférieure à 50 µm.

12. Composition pigmentée selon la revendication 11, dans laquelle le rapport pondéral desdits pigments organiques audit $TiO_2$ pigmentaire rutile est compris entre 20:1 et 1:20.

13. Composition pigmentée selon l'une quelconque des revendications 11 et 12, dans laquelle le rapport en poids dudit dioxyde de titane pigmentaire rutile et desdits pigments organiques à ladite alumine sublimée dans ladite composition est supérieur à 1:1 (par exemple, de 100:1 à 1:1, de 50:1 à 1:1, de 30:1 à 1:1, de 100:1 à 2:1, de 50:1 à 2:1, de 30:1 à 2:1, de 50:1 à 1:1, de 100:1 à 5:1, de 50:1 à 5:1, de 30:1 à 5:1).

14. Procédé de coloration d'un tégument humain comprenant l'application audit tégument humain d'une composition selon l'une quelconque des revendications 1 à 13.

15. Procédé selon la revendication 14, dans lequel ledit tégument humain est les cheveux, la peau, les lèvres ou les ongles.

FIG. 1

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014158599 A2
- US 20070020209 A1
- US 4795631 A
- WO 2019241427 A1
- EP 2724985 A1
- US 8580283 B

**Non-patent literature cited in the description**

- INCI Ingredient Dictionary and Handbook. 2008
- INCI and Handbook. 2008